# EUROPEAN PATENT APPLICATION

(11) **EP 4 778 386 A1**
(43) Date of publication of application: **22.07.2026**
(21) Application number: 24865792.6
(22) Date of filing: 09.09.2024
(51) Int. Cl.: A01G 31/02, A01G 7/06, A01G 9/24, G01N 33/18, A01G 9/26, G01D 21/02, G06N 3/08

(54) **METHOD FOR INTEGRATED CONTROL OF NON-STIRRING HYDROPONIC NUTRIENT SOLUTION SUPPLY SYSTEM**

(30) Priority: 12.09.2023 KR 20230121247
(71) Applicant: Dohwa Engineering Co., Ltd., Seoul 06178 (KR)
(72) Inventor: LEE, Kyeong Il, Paju-si, Gyeonggi-do 10903 (KR); NAM, Woo Sung, Yongin-si, Gyeonggi-do 16871 (KR); HONG, Seongwon, Gimpo-si, Gyeonggi-do 10073 (KR); JE, Sanghee, Hwaseong-si, Gyeonggi-do 18501 (KR)
(74) Representative: Regimbeau
(86) International application number: PCT/KR2024/013619
(87) International publication number: WO 2025/058353

(57) **Abstract**

Provided is an integrated control method for a non-agitator-type hydroponic nutrient solution supply system which includes a raw water tank, a nutrient solution tank and a regulation tank in which raw water is introduced from the raw water tank to generate a nutrient solution and the nutrient solution is stored, and a mixing tank into which raw water is introduced from the raw water tank and the nutrient solution is further introduced from the nutrient solution tank and the regulation tank and the raw water and the nutrient solution are mixed with each other and stored and in which the nutrient solution is supplied by a supply pump 401 from the mixing tank to a growing house GH of a hydroponic facility, the integrated control method including: providing the mixing tank including a nutrient solution recirculation line and a pH sensor; providing valves V1 and V2 in the nutrient solution recirculation line; generating a learning model by setting a mixing tank pH checked by the pH sensor, a mixing tank pH hourly change rate checked by the pH sensor, and recirculation power of the supply pump 401 as a set of learning data; and checking the recirculation power of the supply pump 401 by inputting the mixing tank pH and the mixing tank pH hourly change rate to the generated learning model, so that the supply pump 401 is controlled, and when the recirculation power of the supply pump 401 exceeds 0, the valves V1 and V2 are controlled to open.

## Description

### [Technical Field]

The present disclosure relates to convergence technology of water treatment, agriculture, and information technology (IT).

### [Background Art]

The quality of nutrient solutions used in hydroponics has a significant impact on crop yield. For enhancement and maintenance of nutrient solution quality, in hydroponics, nutrient solutions are formed, managed and supplied in various ways, and in most cases, one mixing tank is placed just before supplying nutrient solutions to a cultivation site for management. This is because checking the pH, electrical conductivity (EC), water level, and temperature of nutrient solutions within one mixing tank and managing them accordingly, information about nutrient solutions supplied to the cultivation site can be obtained and the quality thereof can be maintained.

To this end, various sensors such as a pH sensor, an EC sensor, a water level sensor, a temperature sensor, and the like are provided in the mixing tank of a hydroponic nutrient solution supply system. The pH used in hydroponics is generally about 5.5, and when the pH of the nutrient solution within the mixing tank measured by the pH sensor is lower than 5.5, the pH is adjusted by adding chemicals to a nutrient solution to be supplied to lower the pH, and when the pH of the nutrient solution within the mixing tank measured by the pH sensor is higher than 5.5, the pH is adjusted by further supplying raw water. To this end, an inverter pump is provided in a pipe connecting the mixing tank to each of a nutrient solution supply unit and a raw water tank in which chemicals are stored, and the inverter pump is configured to be manually or automatically controlled based on the pH sensor.

In addition, the total volume can be controlled simultaneously with pH adjustment. For example, when the water level sensor of the mixing tank drops, the inverter pump is operated to introduce chemicals or raw water according to values detected by the pH sensor. When the pH is at an appropriate level, a nutrient solution or a combination of the nutrient solution and raw water is supplied to the mixing tank accordingly.

In this manner, EC, temperature, etc., are also managed.

Meanwhile, independently of this process, an agitator is installed within the mixing tank and operates continuously for 24 hours. Since the nutrient solution in the mixing tank is finally raw water mixed with chemicals, this is intended to manage quality by preventing precipitation of the chemicals and maintaining pH.

Since mixing tanks, which collect and supply nutrient solutions in one place, are typically large-capacity, high-output agitators rated at 11 kW or more are often adopted, depending on the total capacity of the mixing tank. However, when a high-output agitator operates continuously for 24 hours, while precipitation is prevented, a pH variation range and a hourly change rate within the mixing tank may increase. When the pH variation range and the hourly change rate are large, a problem that nutrient solutions harmful to crops are supplied depending on time at which nutrient solutions are supplied to the crops, occurs. This has a negative impact on crop yields.

In addition, when the pH variation range and the hourly change rate are large, driving signals for the nutrient solution supply unit and a raw water tank inverter pump, which are used to regulate the pH, cannot be provided at the appropriate time. For example, when the pH is rapidly changing between 5.4 and 5.8, raw water or chemicals may be supplied depending on the sensing timing of a sensor, so that it becomes more difficult to adjust a nutrient solution to a desired pH.

Furthermore, the RPM of agitators in hydroponic nutrient solution mixing tanks currently in operation is set quite high. In the field, it is common for agitators to operate at up to 180 RPM for 24 hours. While this setting is intended to reliably prevent precipitation, the agitator is unnecessarily rotated at a high rotation speed, and the variation range of the pH further increases.

Furthermore, since unnecessary rotation consumes more energy, this places a significant burden on small-scale farms.

In this way, agitators in mixing tanks for hydroponic nutrient solution supply according to the related art are set to operate continuously, thereby causing problems not only with crop yields but also with maintaining an appropriate pH and energy consumption.
(Patent Document 1) Korean Patent Registration No. 2078056
(Patent Document 2) Korean Patent Registration No. 2079294
(Patent Document 3) Korean Patent Registration No. 2281447
(Patent Document 4) Korean Patent Registration No. 2367100
(Patent Document 5) Korean Patent Registration No. 2541371
(Patent Document 6) Korean Patent Registration No. 2544547
(Patent Document 7) Korean Patent Laid-open Publication No. 2014-0071774
(Patent Document 8) Korean Patent Laid-open Publication No. 2018-0018018
(Patent Document 9) Korean Patent Laid-open Publication No. 2023-0016106
(Patent Document 10) Korean Utility-model Registration No. 20-0248010

### [Disclosure]

### [Technical Problem]

The present disclosure was provided to address the above-described problems.

The present disclosure aims to solve the problems of an increase in the pH variation range, difficulty in maintaining pH levels, and excess energy consumption caused by an agitator that operates at excessive speeds within a mixing tank.

### [Technical Solution]

According to an aspect of the present disclosure, there is provided an integrated control method for a non-agitator-type hydroponic nutrient solution supply system which includes a raw water tank, a nutrient solution tank and a regulation tank in which raw water is introduced from the raw water tank to generate a nutrient solution and the nutrient solution is stored, and a mixing tank into which raw water is introduced from the raw water tank and the nutrient solution is further introduced from the nutrient solution tank and the regulation tank and the raw water and the nutrient solution are mixed with each other and stored and in which the nutrient solution is supplied by a supply pump 401 from the mixing tank to a growing house GH of a hydroponic facility, the integrated control method including: providing the mixing tank including a nutrient solution recirculation line and a pH sensor; providing valves V1 and V2 in the nutrient solution recirculation line; generating a learning model by setting a mixing tank pH checked by the pH sensor, a mixing tank pH hourly change rate checked by the pH sensor, and recirculation power of the supply pump 401 as a set of learning data; and checking the recirculation power of the supply pump 401 by inputting the mixing tank pH and the mixing tank pH hourly change rate to the generated learning model, so that the supply pump 401 is controlled, and when the recirculation power of the supply pump 401 exceeds 0, the valves V1 and V2 are controlled to open.

In addition, an electrical conductivity (EC) sensor may be further provided in the mixing tank, and the set of learning data may further include mixing tank EC checked by the EC sensor and a mixing tank EC hourly change rate checked by the EC sensor, and the recirculation power of the supply pump 401 may be checked by further inputting the mixing EC and the mixing tank EC hourly change rate to the generated learning model.

In addition, the learning model may be a model that outputs the recirculation power of the supply pump 401 that minimizes the mixing tank pH hourly change rate and allows the mixing tank pH to reach predetermined pH by learning mixing tank pH checked by the pH sensor, a mixing tank pH hourly change rate checked by the pH sensor, mixing tank EC checked by the EC sensor, a mixing tank EC hourly change rate checked by the EC sensor, and the recirculation power of the supply pump 401.

In addition, a CO₂ tank, a water level sensor, and a temperature sensor may be further provided in the mixing tank, and the set of learning data may further include a mixing tank water level checked by the water level sensor and mixing tank temperature checked by the temperature sensor.

In addition, the nutrient solution tank may allow the nutrient solution to be introduced into the mixing tank by a nutrient solution dosing pump, the regulation tank may allow the nutrient solution to be introduced into the mixing tank by a regulation pump, and the mixing tank water level and the mixing tank temperature may be further input to the generated learning model so that power of the nutrient solution dosing pump and power of the regulation pump may be further checked as well as the recirculation power of the supply pump 401.

In addition, a humidity sensor and a moisture content sensor may be provided in the growing house GH, and the set of learning data may further include humidity in the growing house checked by the humidity sensor and a moisture content in the growing house checked by the moisture content sensor.

In addition, a CO₂ tank may be further provided in the growing house GH, and the humidity in the growing house and the moisture content in the growing house may be further input to the generated learning model so that a CO₂ emission rate of the CO₂ tank may be further checked as well as recirculation power of the supply pump 401, power of the nutrient solution dosing pump, and power of the regulation pump.

In addition, by the integrated control method, the mixing tank pH hourly change rate may be reduced compared to when a mixing tank agitator within the mixing tank iscontinuously operated.

In addition, the nutrient solution tanks may be two or more, each nutrient solution tank
may be equipped with the nutrient solution dosing pump, and a maximum output of the nutrient solution dosing pump may be greater than a maximum output of the regulation pump.

In addition, each nutrient solution tank may be equipped with a nutrient solution tank agitator, the regulation tank may be equipped with a regulation tank agitator, and a maximum output of the nutrient solution tank agitator may be greater than a maximum output of the regulation tank agitator.

According to another aspect of the present disclosure, there is provided a computer program stored on a computer-readable recording medium so that the above-described methods may be performed.

### [Advantageous Effects]

According to the present disclosure, a hydroponic nutrient solution supply system that prevents precipitation without using an agitator in a mixing tank is provided. In particular, the amount of recirculation can be controlled by artificial intelligence (AI) by utilizing various sensor values detected in the mixing tank. As a result, unlike an agitator according to the related art that operates continuously for 24 hours, problems such as a large variation range in a RPM of a nutrient solution or a significant hourly change rate can be fundamentally prevented. This may lead to an increase in crop yields.

Since an agitator itself is not used, this method has energy-saving effects.

In another embodiment of the present disclosure, volumes of nutrient solutions supplied from a nutrient solution tank and a regulation tank can be simultaneously controlled, as well as automatically regulate the CO₂ emission rate from a CO₂ tank in a cultivation area. Thus, most of devices in the hydroponic nutrient solution supply system can be integrated and automatically controlled, and in this case, a pH variation range and a hourly change rate can be more precisely controlled.

### DESCRIPTION OF THE DRAWINGS

FIG. 1 is a view illustrating a non-agitator-type hydroponic nutrient solution supply system according to the present disclosure; and
FIGS. 2 and 3 are views illustrating an integrated control method according to the present disclosure.

### [Brief Description of the Drawing]

### 1. Description of non-agitator-type hydroponic nutrient solution supply system

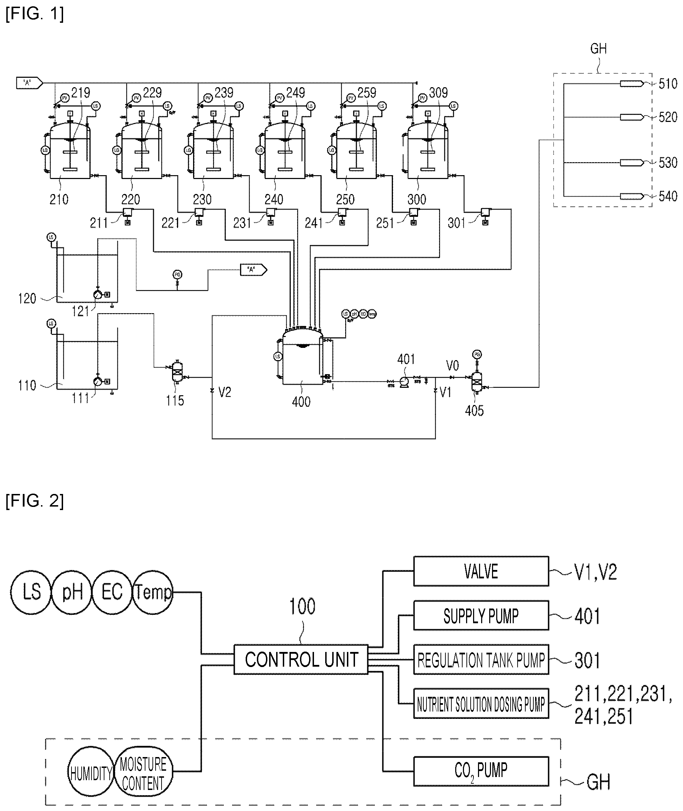

A non-agitator-type hydroponic nutrient solution supply system to which a method
according to the present disclosure is applied, will be described with reference to FIG. 1.

The hydroponic nutrient solution supply system includes raw water tanks 110 and 120, nutrient solution tanks 210, 220, 230, 240, and 250, a regulation tank 300, and a mixing tank 400.

The raw water tanks 110 and 120 store raw water to be supplied to the nutrient solution tanks 210, 220, 230, 240, and 250, the regulation tank 300, and the mixing tank 400. Raw water is supplied from one raw water tank 110 to the mixing tank 400, and raw water is supplied from another raw water tank 120 to the nutrient solution tanks 210, 220, 230, 240, and 250 (labeled as "A" on the drawings). Although FIG. 1 shows the hydroponic nutrient solution supply system divided into two nutrient solution tanks, the number of tanks is not limited. For example, one raw water tank may supply raw water to the mixing tank, the nutrient solution tanks, and the regulation tank, or three or more raw water tanks may be provided and supply raw water to each of the mixing tank, the nutrient solution tanks, and the regulation tanks.

A raw water feed pump 111 is provided inside the raw water tank 110. When the raw water feed pump 111 operates, raw water is supplied to the mixing tank 400. A filtration unit 115 may be located in a corresponding flow path. The filtration unit 115 may be, for example, a cartridge filter.

Similarly, a raw water feed pump 121 is provided inside the raw water tank 120. When the raw water feed pump 121 operates, raw water is supplied to the nutrient solution tanks 210, 220, 230, 240, and 250, and the regulation tank 300.

Each of the raw water tanks 110 and 120 may be connected to ground water to receive raw water directly, and raw water flown through each of the raw water tanks 110 and 120 may be configured to flow underground.

Each of the raw water tanks 110 and 120 is provided with a diameter of 5.35 m and a height of 3.5 m, for example, and is capable of storing 60.7 m³ of raw water, but the present disclosure is not limited thereto.

The nutrient solution tanks 210, 220, 230, 240, and 250 are provided in plurality. Although there are five nutrient solution tanks on the drawings, the present disclosure is not limited thereto.

Raw water is supplied from the raw water tank 120 to each of the nutrient solution tanks 210, 220, 230, 240, and 250 in a state where separate chemicals for generating nutrient solutions are supplied (not shown), so that a nutrient solution suitable for hydroponic cultivation may be generated and stored. To this end, an equal amount of raw water is supplied to each of the nutrient solution tanks 210, 220, 230, 240, and 250.

The chemicals used for generating nutrient solutions are not limited, and the type of the chemicals may be changed according to crops. Thus, preferable pH values of nutrient solutions may be changed. Although it is described that a pH of 5.5 is preferable, the present disclosure is not limited thereto.

Each of the nutrient solution tanks 210, 220, 230, 240, and 250 includes each of nutrient solution tank agitators 219, 229, 239, 249, and 259 that continuously agitates the incoming chemicals and raw water. These may be, for example, paddle-type agitators operating at 180 RPM and 1.5 kW, but the present disclosure is not limited thereto.

In addition, a water level sensor LS and a water level gauge LG are provided in each of the nutrient solution tanks 210, 220, 230, 240, and 250. Each of the nutrient solution tanks 210, 220, 230, 240, and 250 independently checks the water level of the nutrient solution using the water level sensor, and when it is checked that the nutrient solution is stored below a certain amount, a valve PV opens, and raw water is introduced from the raw water tank 120. In addition, the current water level is checked with the naked eye using the water level gauge LG.

In addition, each of the nutrient solution tanks 210, 220, 230, 240, and 250 includes nutrient solution dosing pumps 211, 221, 231, 241, and 251 for supplying nutrient solutions to the mixing tank 400. The nutrient solution dosing pumps 211, 221, 231, 241, and 251 may be precisely controlled by an inverter motor. The nutrient solution dosing pumps 211, 221, 231, 241, and 251 may have a capacity of, for example, 8.8 L/min x 30 mH and a power rating of 0.01 kW, but the present disclosure is not limited thereto.

In this way, each of the nutrient solution tanks 210, 220, 230, 240, and 250 stores a certain amount of nutrient solution constantly. However, the pH and EC of this nutrient solution have not been precisely adjusted, and this is done by the regulation tank 300 and the mixing tank 400.

Similar to the nutrient solution tanks 210, 220, 230, 240, and 250, the regulation tank 300 generates and stores a nutrient solution by receiving raw water from the raw water tank 120 in a state where separate chemicals are supplied (not shown), and unlike the nutrient solution tanks 210, 220, 230, 240, and 250, the regulation tank 300 performs the function of precisely adjusting the total volume, pH, electrical conductivity (EC), and etc. of the nutrient solution in the mixing tank 400, which contains the final form of nutrient solution supplied to a growing house GH.

Similar to the nutrient solution tanks 210, 220, 230, 240, and 250, the regulation tank 300 also includes a regulation tank pump 301 and a regulation tank agitator 309, however, after a large amount of a nutrient solution is supplied from the nutrient solution tanks 210, 220, 230, 240, and 250 to the mixing tank 400, the volume of the nutrient solution from the regulation tank 300 is precisely controlled and supplied so that the volume, pH, EC, etc. of the nutrient solution in the mixing tank 400 are adjusted and thus, the regulation tank pump 301 and the regulation tank agitator 309 need to be more precisely controlled. Thus, it is preferable to use devices that are compact and precisely controlled rather than large-capacity devices. In other words, it is preferable that the regulation tank pump 301 has a lower maximum output than the nutrient solution dosing pumps 211, 221, 231, 241, and 251, and it is preferable that the regulation tank agitator 309 also has a lower maximum output than the nutrient solution tank agitators 219, 229, 239, 249, and 259. For example, the regulation tank pump 301 may have a capacity of 0.1 L/min x 30 mH and a power rating of 0.01 kW, and the regulation tank agitator 309 may be a paddle-type agitator operating at 30 RPM and 1.5 kW, but the present disclosure is not limited thereto.

The mixing tank 400 is a final tank that stores a nutrient solution by receiving the nutrient solution from the nutrient solution tanks 210, 220, 230, 240, and 250 and the regulation tank 300, and when necessary, the mixing tank 400 allows a certain amount of the nutrient solution to be transferred to the growing house GH and discharged through nutrient solution discharge units 510, 520, 530, and 540 in the growing house. To this end, a valve V0, a supply pump 401, and a filtration unit 415 are provided downstream. Although, on the drawings, the nutrient solution being supplied in four branches, the present disclosure is not limited thereto.

Since the nutrient solution in the mixing tank 400 is a final nutrient solution supplied to the growing house GH, the pH, EC, temperature, etc. of the nutrient solution need to be precisely adjusted. After a certain amount of nutrient solution has been supplied from the nutrient solution tanks 210, 220, 230, 240, and 250, the pH, EC, temperature, etc. of the nutrient solution in the mixing tank 400 are controlled by regulating the nutrient solution supplied from the regulation tank 300.

To this end, the mixing tank 400 includes a pH sensor pH, an EC sensor EC, a water level sensor LS, and a temperature sensor Temp, etc.. In addition, the water level gauge LG is further provided and may be checked with an operator's naked eye.

In the present disclosure, a mixing tank agitator is not provided in the mixing tank 400. A recirculation flow path is provided to perform the function of preventing precipitation of the mixing tank agitator. That is, precipitation is prevented by ensuring that the nutrient solution in the mixing tank 400 is recirculated at an appropriate time and in an appropriate amount. The timing and amount of recirculation are determined by an artificial intelligence learning model described later.

Recirculation is carried out along a nutrient solution recirculation path and is made possible by controlling the above-described supply pump 401 and two valves V1 and V2. When the valves V1 and V2 are opened and the supply pump 401 operates at the time of recirculation, the nutrient solution recirculates. That is, when the supply pump 401 operates, the nutrient solution may be supplied to the growing house GH or may recirculate depending on the operation of the valves V0, V1, and V2, and the power of the supply pump 401 during nutrient solution recirculation is referred to as "recirculation power".

When the nutrient solution is supplied into the mixing tank 400 through recirculation, turbulence is formed, and at the same time, chemicals that are partially settled or not sufficiently dissolved are dissolved, thereby adjusting the pH, EC, temperature, etc.

However, in this case, since the range of pH control is not large, when a larger pH control value is required, the pH may be changed by controlling the regulation tank 300, or the pH may be changed significantly at the same time as the nutrient solution recirculation by using a pH control chemical storage tank (not shown) separately provided in a recirculation valve.

As described above in the related art, mixing tank agitators according to the related art are high capacity units rated at 11 kW or more and operate continuously 24 hours a day. The mixing tank agitators according to the related art rotate at maximum speed at all times to prevent precipitation, and in this case, the variation range of pH and a hourly change rate are large, which adversely affect crop yields.

In the present disclosure, it is possible to prevent precipitation without adopting the mixing tank agitator at all. Furthermore, a control unit 100 precisely controls the amount of the recirculating nutrient solution automatically by using Al-based learning models. This will be described later.

The growing house GH may be any type of hydroponic facility.

The growing house GH is equipped with a plurality of growing media for crops, as well as a humidity sensor and a moisture content sensor. Since the humidity sensor has a probe inserted into the growing media in the growing house GH, hereinafter, the humidity of the growing house GH refers to the humidity of the growing media. The moisture content sensor in the growing house GH checks the moisture content of the air inside the facility of the growing house GH.

### 2. Description of integrated control method

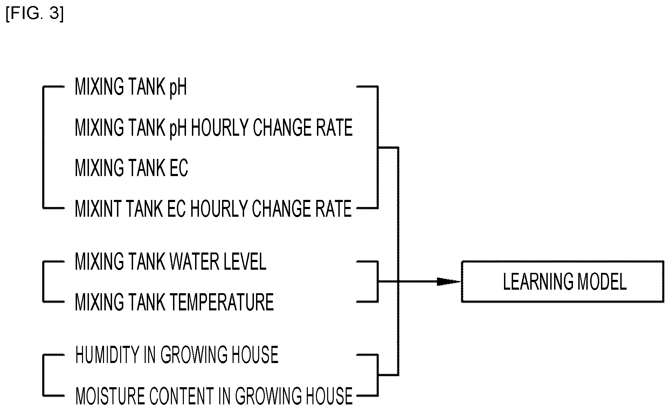

An integrated control method according to the present disclosure will be described with reference to FIGS. 2 and 3.

The control unit 100 according to the present disclosure constructs learning models using learning data and then performs integrated control using the learning models.

The control unit 100 checks the pH and EC of the nutrient solution stored in the mixing tank 400 through the pH sensor and the EC sensor in the mixing tank 400. In another embodiment, the temperature and water level are further checked through the temperature sensor and the water level sensor in the mixing tank 400. In another embodiment, humidity and moisture content are further checked through the humidity sensor and the moisture content sensor in the growing house GH.

In addition, the control unit 100 checks recirculation power of the supply pump
401. In another embodiment, the control unit 100 checks the power of the regulation tank pump 301 and the power of the nutrient solution dosing pumps 211, 221, 231, 241, and 251. In another embodiment, the CO₂ emission rate of the CO₂ tank in the growing house GH is further checked.

The control unit 100 sets learning data using them.

Specifically, a plurality of sets of learning data are collected by setting the pH of the nutrient solution in the mixing tank 400 (hereinafter referred to as "mixing tank pH"), the hourly change rate in pH (hereinafter referred to as a "mixing tank pH hourly change rate"), and the recirculation power of the supply pump 401 as learning data. To acquire the learning data, it is preferable to vary the recirculation power of the supply pump 401 widely. In any situation, as the recirculation power of the supply pump 401 changes, the nutrient solution that recirculates changes, and the pH and the hourly change rate in pH, which are causally related, will also change.

After acquiring the learning data, input values are set to the mixing tank pH and the mixing tank pH hourly change rate, and an output value is set to the recirculation power of the supply pump 401. At this time, an output value that minimizes the mixing tank pH hourly change rate while ensuring that the mixing tank pH approaches a preferable value, specifically 5.5, is calculated. A learning model is generated in such a way that, when specific mixing tank pH and the mixing tank pH hourly change rate are input through iterative learning, the recirculation power of the supply pump 401 that minimizes the mixing tank pH hourly change rate while ensuring the mixing tank pH reaches 5.5, is output.

After the learning data is generated, the learning data is applied to the hydroponic nutrient solution supply system. The control unit 100 senses the mixing tank pH and the mixing tank pH hourly change rate in real time and applies them to the learning model, and thus, the appropriate recirculation power of the supply pump 401 is output. The supply pump 401 operates depending on the output value. When the supply pump 401 operates for recirculation, i.e., when the recirculation power of the supply pump 401 exceeds 0, the valves V0, V1, and V2 operate accordingly. The valve V0 will be closed, and the valves V1 and V2 in a recirculation line will be opened. As occasion demands, chemicals etc. may be additionally supplied to the recirculation line, or the regulation tank 300 may operate together.

As recirculation is performed, the mixing tank pH and the mixing tank pH hourly change rate begin to vary in real time. After a certain period (e.g., 1 minute) has elapsed, the changed mixing tank pH and mixing tank pH hourly change rate are fed back into the learning model of the control unit 100, so that new recirculation power of the supply pump 401 is output again. Recirculation by the supply pump 401 is performed with the output value.

In this manner, the recirculation power of the supply pump 401 continues to change dynamically, and recirculation may be performed or not, and even when recirculation is performed, amounts of recirculating nutrient solutions are different from each other. By using this, the pH may be maintained at 5.5 while minimizing the mixing tank pH hourly change rate.

In an embodiment of the present disclosure, the EC of the nutrient solution in the mixing tank 400 (hereinafter referred to as mixing tank EC) and the EC hourly change rate (hereinafter referred to as mixing tank EC hourly change rate) may be further used as a set of learning data. In this case, it is more preferable to ensure that the pH of the nutrient solution within the mixing tank 400 reaches the desired level while minimizing not only the mixing tank pH hourly change rate but also the mixing tank EC hourly change rate.

In another embodiment of the present disclosure, the temperature and water level detected by the temperature sensor and water level sensor in the mixing tank 400, as well as the power of the nutrient solution dosing pumps 211, 221, 231, 241, and 251 and the power of the control pump 301, may be further set as learning data. This is because the temperature and water level of the nutrient solution are related to the amount of nutrient solution flowing into the nutrient solution tanks 210, 220, 230, 240, and 250 and the regulation tank 300, and simultaneously affect the pH and EC of the mixing tank 400. Through the present embodiment, the inflow volume of the nutrient solution tanks 210, 220, 230, 240, and 250 and the regulation tank 300 is automatically controlled.

In another embodiment of the present disclosure, the humidity and moisture content in the growing house GH and the CO₂ emission rate from the CO₂ tank in the growing house GH may be further set as learning data. This is because the humidity and moisture content in the growing house GH are correlated with the CO₂ emission rate from the CO₂ tank, and at the same time, the humidity and moisture content in the growing house GH are also influenced by the pH and EC of the nutrient solution. The present embodiment provides the effect of automatically controlling the CO₂ tank in the growing house GH.

### [Explanation of reference numerals]

100: control unit
110, 120: raw water tank
111, 121: raw water feed pump
115, 405: filtration unit
210, 220, 230, 240, 250: nutrient solution tank
211, 221, 231, 241, 251: nutrient solution dosing pump
219, 229, 239, 249, 259: nutrient solution tank agitator
300: regulation tank
301: regulation tank pump
309: regulation tank agitator
400: mixing tank
401: supply pump
510, 520, 530, 540: nutrient solution discharge units
GH: growing house
LS: water level sensor
LG: water level gauge
pH: pH sensor
EC: EC sensor
Temp: temperature sensor
V0, V1, V2: valve

## Claims

1. An integrated control method for a non-agitator-type hydroponic nutrient solution supply system which comprises a raw water tank, a nutrient solution tank and a regulation tank in which raw water is introduced from the raw water tank to generate a nutrient solution and the nutrient solution is stored, and a mixing tank into which raw water is introduced from the raw water tank and the nutrient solution is further introduced from the nutrient solution tank and the regulation tank and the raw water and the nutrient solution are mixed with each other and stored and in which the nutrient solution is supplied by a supply pump 401 from the mixing tank to a growing house GH of a hydroponic facility, the integrated control method comprising:
providing the mixing tank including a nutrient solution recirculation line and a pH sensor;
providing valves V1 and V2 in the nutrient solution recirculation line;
generating a learning model by setting a mixing tank pH checked by the pH sensor, a
mixing tank pH hourly change rate checked by the pH sensor, and recirculation power of the supply pump 401 as a set of learning data; and
checking the recirculation power of the supply pump 401 by inputting the mixing
tank pH and the mixing tank pH hourly change rate to the generated learning model, so that the supply pump 401 is controlled, and when the recirculation power of the supply pump 401 exceeds 0, the valves V1 and V2 are controlled to open,
wherein an electrical conductivity (EC) sensor is further provided in the mixing tank, and the learning model is a model that outputs the recirculation power of the supply pump 401 that minimizes the mixing tank pH hourly change rate and allows the mixing tank pH to reach predetermined pH by learning mixing tank pH checked by the pH sensor, a mixing tank pH hourly change rate checked by the pH sensor, mixing tank EC checked by the EC sensor, a mixing tank EC hourly change rate checked by the EC sensor, and the recirculation power of the supply pump 401.

2. The integrated control method of claim 1, wherein the set of learning data
further comprises mixing tank EC checked by the EC sensor and a mixing tank EC hourly change rate checked by the EC sensor, and the recirculation power of the supply pump 401 is checked by further inputting the mixing EC and the mixing tank EC hourly change rate to the generated learning model.

3. The integrated control method of claim 1, wherein a CO₂ tank, a water level
sensor, and a temperature sensor are further provided in the mixing tank, and the set of learning data further comprises a mixing tank water level checked by the water level sensor and mixing tank temperature checked by the temperature sensor.

4. The integrated control method of claim 3, wherein the nutrient solution tank
allows the nutrient solution to be introduced into the mixing tank by a nutrient solution dosing pump, the regulation tank allows the nutrient solution to be introduced into the mixing tank by a regulation pump, and the mixing tank water level and the mixing tank temperature are further input to the generated learning model so that power of the nutrient solution dosing pump and power of the regulation pump are further checked as well as the recirculation power of the supply pump 401.

5. The integrated control method of claim 4, wherein a humidity sensor and a
moisture content sensor are provided in the growing house GH, and the set of learning data further comprises humidity in the growing house checked by the humidity sensor and a moisture content in the growing house checked by the moisture content sensor.

6. The integrated control method of claim 5, wherein a CO₂ tank is further
provided in the growing house GH, and the humidity in the growing house and the moisture content in the growing house are further input to the generated learning model so that a CO₂ emission rate of the CO₂ tank is further checked as well as recirculation power of the supply pump 401, power of the nutrient solution dosing pump, and power of the regulation pump.

7. The integrated control method of claim 6, wherein, by the integrated
control method, the mixing tank pH hourly change rate is reduced compared to when a mixing tank agitator within the mixing tank iscontinuously operated.

8. The integrated control method of claim 7, wherein the nutrient solution
tanks are two or more, each nutrient solution tank is equipped with the nutrient solution dosing pump, and a maximum output of the nutrient solution dosing pump is greater than a maximum output of the regulation pump.

9. The integrated control method of claim 8, wherein each nutrient solution
tank is equipped with a nutrient solution tank agitator, the regulation tank is equipped with a regulation tank agitator, and a maximum output of the nutrient solution tank agitator is greater than a maximum output of the regulation tank agitator.

10. A computer program stored on a computer-readable recording medium so
that the integrated control method for the non-agitator-type hydroponic nutrient solution supply system according to one of claims 1 through 9 is performed by a computer.
